# EUROPÄISCHE PATENTSCHRIFT

(11) **EP 0 531 896 B1**
(45) Veröffentlichungstag und Bekanntmachung des Hinweises auf die Patenterteilung: **28.06.1995**
(21) Anmeldenummer: 92115132.0
(22) Anmeldetag: 04.09.1992
(51) Int. Cl.: C09K 3/14, A61K 7/16

(54) **Mittel zur Oberflächenbehandlung von Dentalteilen, insbesondere Schleif-, Polier- und/oder Strahlmittel**
Means for the superficial treatment of dental pieces, in particulier for abrading, polishing and/or buffing
Moyen de traitement superficiel de pièces dentaires, en particulier pour la meulage, le polissage et/ou le nettoyage abrasif

(30) Priorität: 07.09.1991 DE 4129870; 07.11.1991 DE 4136592
(43) Veröffentlichungstag der Anmeldung: 17.03.1993
(73) Patentinhaber: BEGO Bremer Goldschlägerei Wilh. Herbst GmbH & Co., 28329 Bremen (DE)
(72) Erfinder: Gundlach, Hans-Werner, Dr.-Ing., W-2800 Bremen 1 (DE); Stroncik, Peter, W-2860 Osterholz-Scharmbeck (DE)
(74) Vertreter: Möller, Friedrich, Dipl.-Ing.

(56) Entgegenhaltungen:
- DE-A- 2 747 852
- DATABASE WPIL, Week 8530, Derwent Publications Ltd., London, GB; AN 85-180799; & JP-A-60 108 500
- DATABASE WPIL, Week 9127, Derwent Publications Ltd., London, GB; AN 91-196387; & JP-A-31 20 204

## Beschreibung

Die Erfindung betrifft ein Mittel zur Oberflächenbehandlung von Dentalteilen, insbesondere ein Schleif- und/oder Poliermittel nach dem Oberbegriff des Anspruchs 1 und die besondere Verwendung des Mittels.

Die hier angesprochenen Mittel dienen zur Oberflächenbehandlung von Dentalteilen wie z. B. Zahnersatz, Kronen, Brücken, Modellguß und/oder Verblendkeramiken. Solche Mittel, und zwar insbesondere Schleif-, Polier- und/oder Strahlmittel, enthalten üblicherweise Fest- oder Füllstoffe aus Quarz. Quarz ist ein kristallisiertes Siliciumdioxid. Es hat sich herausgestellt, daß häufig mit Quarz arbeitende Personen an einer typischen Krankheit, der sogenannten Silikose, erkranken. Quarz ist daher gesundheitsgefährdend.

Der Erfindung liegt ausgehend vom Vorstehenden die Aufgabe zugrunde, ein Mittel zur Oberflächenbehandlung von Dentalteilen, insbesondere Schleif-, Polier- und/oder Strahlmittel, zu schaffen, das gesundheitlich unbedenklich ist und gleichwohl im wesentlichen die Eigenschaften der bekannten Mittel dieser Art aufweist.

Ein zur Lösung dieser Aufgabe dienendes Mittel zur Oberflächenbehandlung von Dentalteilen weist die Merkmale des Anspruchs 1 auf. Durch die Bildung der Feststoffe aus einem Pulver eines quarzfreien, magmatischen Gesteins entsteht ein Mittel dieser Art ohne kristallisierte Siliciumoxide, das überraschenderweise die im wesentlichen gleichen Wirkungen zeigt, die mit Schleif-, Polier- oder Strahlmittel erzielt werden, die Fest- oder Füllstoffe aus Quarz enthalten. Diese Mittel, nämlich Schleif-, Polier- und/oder Strahlmittel, eignen sich besonders zur Verwendung bei der Oberflächenbehandlung in der Dentaltechnik, und zwar hier vor allem zum Schleifen, Polieren und/oder Strahlen von Dentalteilen.

Als quarzfreie, magmatische Gesteine kommen in Betracht Foyaite, Phonolithe, Nephelinbasalte und insbesondere Nephelinsyenite. Vor allem die letztgenannten Nephelinsyenite zeigen nahezu die gleichen Schleif-, Polier- oder Strahleigenschaften wie Mittel dieser Art mit Quarz.

Als besonders vorteilhaft hat sich die Verwendung von Nephelinsyeniten mit einer Korngröße von 2 bis 300 »m, insbesondere 60 bis 150 »m, herausgestellt. Vor allem für Schleif-, Polier- und Strahlzwecke zeigen Nephelinsyenite mit einer solchen Korngröße eine sehr gute Wirksamkeit und Standzeit, die reinen kristallisierten Siliciumoxiden, also Quarz, kaum nachstehen.

Nach einer bevorzugten Weiterbildung der Erfindung enthalten insbesondere Schleif- und/oder Poliermittel, bei denen die Nephelinsyenite in einer flüssigen oder pastösen Masse eingelagert sind, ein zusätzliches Bakterizid. Dieses ist zweckmäßigerweise in der flüssigen oder pastösen Masse eingemischt. Hierdurch weist das Mittel desinfizierende Eigenschaften auf. Diese sind insbesondere bei Verwendung des Mittels in der Dentaltechnik von Vorteil.

Nachfolgend wird die Erfindung anhand bevorzugter Beispiele erläutert:
Ein Schleif- bzw. Poliermittel, das sich insbesondere zum Einsatz in Dentallabors und hier vor allem zum Polieren von Kunststoffteilen des Dentalbereichs wie z. B. Dentalprothesen, Kronen, Brücken oder dergleichen eignet, setzt sich wie folgt zusammen:
100 l Wasser
100 kg Glycerin
2 l Entschäumer (z. B. Silikonöl)
1000 kg Nephelinsyenit
0,3 bis 2,5 kg bakterizide Zusätze.

Als bakterizide Zusätze kommen quaternäre Amine, Phenolderivate und/oder Parabene in Betracht. Bei Mitteln, die eine bakterizide Wirkung nicht aufweisen müssen, können die bakteriziden Zusätze entfallen.

Als Strahlmittel wird Nephelinsyenit in körniger bzw. pulvriger Form mit einer Korngröße im Bereich zwischen 2 und 300 »m verwendet. Es ist dabei aus dem vorstehend angegebenen Korngrößenbereich jeweils diejenige Korngröße oder ein Teilspektrum aus dem Korngrößenbereich auszuwählen, daß dem zu behandelnden Material oder der gewünschten Oberflächengüte entspricht. Es ist auch denkbar, ein Gemisch von Nephelinsyenit unterschiedlicher Korngröße bzw. Korngrößengruppen als Strahlmittel zu verwenden.

Als Nephelinsyenit der hier angesprochenen Art kommt in Betracht
NaAlSiO₄, KAlSi₃O₈
oder
NaAlSiO₄, (Na,Ca) AlSi₃O₈
Das Nephelinsyenit liegt in den vorstehend angegebenen Beispielen als Pulver mit einer Korngröße von 2 bis 300 »m, insbesondere 60 bis 150 »m, vor. Die Farbe entspricht der Naturfarbe. Es ist auch denkbar, Mittel nach den obigen Beispielen herzustellen, bei denen das Nephelinsyenit ersetzt ist durch Foyaite, Phonolithe oder Nephelinbasalte in gleicher Menge und im gleichen Korngrößenbereich.

## Patentansprüche

1. Mittel zur Oberflächenbehandlung, insbesondere Schleif- und/oder Poliermittel, mit in einer vorzugsweise flüssigen oder pastösen Masse eingelagerten Feststoffen aus einem Nephelinsyenit, **gekennzeichnet durch** ein feinkörniges bzw. pulvriges Nephelinsyenit mit einer Korngröße von 2 bis 300 »m, und ein Bakterizid.

2. Mittel nach Anspruch 1, dadurch gekennzeichnet, daß das Bakterizid der flüssigen oder pastösen Masse zugeordnet ist.

3. Verwendung des Mittels nach Anspruch 1 oder 2 zur Oberflächenbehandlung von Dentalteilen, insbesondere zum Schleifen und/oder Polieren von Dentalteilen wie vorzugsweise Zahnersatz, Kronen, Brücken, Modellguß und/oder Verblendkeramiken.

## Claims

1. Material for surface treatment, especially grinding and/or polishing material, with solids consisting of a nepheline syenite which are included in a preferably liquid or pasty mass, characterized by a fine-granular or pulverulent nepheline syenite with a grain size of 2 to 300 »m and by a bactericide.

2. Material according to Claim 1, characterized in that the bactericide is assigned to the liquid or pasty mass.

3. Use of the material according to Claim 1 or 2 for the surface treatment of dental parts, especially for the grinding and/or polishing of dental parts, such as preferably dental prostheses, crowns, bridges, model castings and/or facing ceramics.

## Revendications

1. Produit de traitement de surface, en particulier produit de meulage et/ou de polissage avec des solides inclus dans une masse de préférence liquide ou pâteuse et constitués d'une néphélinyénite, caractérisé par le fait que la néphélinyénite, qui a une granulométrie fine ou qui se présente sous forme pulvérulente, a une taille de grain comprise dans la plage allant de 2 à 300 »m et contient un bactéricide.

2. Produit selon la revendication 1, caractérisé en ce que le bactéricide est ajouté à la masse liquide ou pâteuse.

3. Utilisation du produit selon la revendication 1 ou 2, pour le traitement de surface de parties dentaires, en particulier pour meuler et/ou polir des parties dentaires telles que par exemple un dentier, des couronnes, des bridges, des pièces moulées au modèle et/ou des céramiques de masquage.
